# EUROPÄISCHE PATENTSCHRIFT

(11) **EP 2 743 247 B1**
(45) Veröffentlichungstag und Bekanntmachung des Hinweises auf die Patenterteilung: **11.01.2017**
(21) Anmeldenummer: 13194557.8
(22) Anmeldetag: 27.11.2013
(51) Int. Cl.: C07C 29/50, C07C 45/00, C07C 45/33, C07C 45/82, C07C 35/205, C07C 49/413, C07D 225/02, C07C 29/80

(54) **Aufarbeitung eines CDON/CDOL-Gemisches mittels einer Sequenz von Seitenabzugskolonnen**
The processing of a CDON/CDOL mixture by means of a sequence of side withdrawal columns
Traitement d'un mélange CDON/CDOL au moyen d'une séquence de colonnes à sortie latérale

(30) Priorität: 17.12.2012 DE 102012223370
(43) Veröffentlichungstag der Anmeldung: 18.06.2014
(73) Patentinhaber: Evonik Degussa GmbH, 45128 Essen (DE)
(72) Erfinder: Cameretti, Luca, 44267 Dortmund (DE); Demicoli, Daniel, 45131 Essen (DE); Meier, Ralf, 44265 Dortmund (DE)

(56) Entgegenhaltungen:
- DE-A1-102005 048 250

## Beschreibung

Die Erfindung betrifft ein Verfahren zur Abtrennung einer Cyclododecanon-reichen Fraktion aus einem Leichtsieder, Cyclododecanon, Mittelsieder, Cyclododecanol und Schwersieder enthaltenden Dehydrierungsgemisch.

Butadien wird im Folgenden als Kurzwort für die Substanz 1,3-Butadien, CAS-Nr. 106-99-0 verwendet.

CDT wird im Folgenden als Abkürzung verwendet für die Substanz 1,5,9-Cyclododecatrien, CAS-Nr. 4904-61-4.

CDEN wird im Folgenden als Abkürzung verwendet für die Substanz Cyclododecen, CAS-Nr. 1501-82-2.

CDAN wird im Folgenden als Abkürzung verwendet für die Substanz Cyclododecan, CAS-Nr. 294-62-2.

CDON im Folgenden als Abkürzung verwendet für die Substanz Cyclododecanon, CAS-Nr. 830-13-7.

CDOL wird im Folgenden als Abkürzung verwendet für die Substanz Cyclododecanol, CAS-Nr. 1724-39-6.

CDOL t.q. steht für CDOL in technischer Qualität und bezeichnet ein Gemisch enthaltend 75 bis 85 Gew.-% CDOL und 10 bis 20 Gew.-% CDON.

Oxim wird im Folgenden als Kurzwort gebraucht für das Oxim des CDON, CAS-Nr. 9466-89-4.

Laurinlactam ist der Trivialname von Azacyclotridecan-2-on, CAS-Nr. 947-04-6.

Laurinlactam ist der Ausgangsstoff für die Herstellung des Hochleistungskunststoffes Polyamid 12. Laurinlactam ist im industriellen Maßstab über die folgende Route erhältlich: Das bei der Erdölverarbeitung anfallende Butadien wird mittels katalytischer Cyclotrimerisierung in CDT umgesetzt. Durch Hydrierung des CDT entsteht CDAN. Die Oxidation des CDAN mit (Luft-) Sauerstoff ergibt ein Gemisch aus CDOL und CDON. Dieses Gemisch wird einer Dehydrierung unterworfen, welche das in dem Gemisch enthaltene CDOL in CDON umsetzt. Es fällt ein Dehydrierungsgemisch an, welches hauptsächlich CDON enthält. Daneben umfasst das Dehydrierungsgemisch nicht umgesetztes CDOL und weitere Komponenten. Hochreines CDON wird aus dem Dehydrierungsgemisch abgetrennt. Das hochreine CDON wird zu seinem Oxim oximiert. Das Oxim wird anschließend mit Schwefelsäure zu Laurinlactam umgesetzt.

Der gesamte Prozess ist mit weiteren Nachweisen beschrieben in Oenbrink, G. and Schiffer, T. 2009. Cyclododecanol, Cyclododecanone, and Laurolactam. Ullmann's Encyclopedia of Industrial Chemistry. DOI: 10.1002/14356007.a08_201.pub2

Die Erfindung befasst sich mit der Aufarbeitung des CDON / CDOL-haltigen Dehydrierungsgemisches mit dem Ziel, daraus hochreines CDON zu gewinnen.

Das auf der oben beschriebenen Route anfallende Dehydrierungsgemisch enthält neben CDON und CDOL weitere Komponenten in Gestalt von Leichtsiedern, Mittelsiedern und Schwersiedern.

"Leichtsieder" im Sinne dieser Erfindung sind Stoffe oder Stoffgemische, die bei denselben Druckbedingungen einen niedrigeren Siedepunkt als CDON aufweisen und sich daher bei der destillativen Trennung eines Gemisches aus Leichtsiedern und CDON im Destillat anreichern. Die wesentlichen Leichtsieder in diesem Zusammenhang sind: Cyclododecen (CDEN), Cyclododecan (CDAN), Dodecanal, Cyclododecan-Epoxid. Cyclododecan-Epoxid liegt auf der Grenze der obigen Definition der Leichtsieder, da sein Siedepunkt von etwa 150°C bei 40 mbar praktisch dem des CDON entspricht und daher von dem CDON nicht wirtschaftlich zu trennen ist. In geringeren Mengen (kleiner 100 ppm) kommen noch die Leichtsieder Essigsäure und Decan vor, diese sind aber für die Trennaufgaben kaum relevant.

"Mittelsieder" im Sinne dieser Erfindung sind Stoffe oder Stoffgemische, die bei denselben Druckbedingungen einen höheren Siedepunkt als CDON und einen niedrigeren Siedepunkt als CDOL aufweisen und sich daher bei der destillativen Trennung eines Gemisches aus CDON, Mittelsiedern und CDOL in der Mitte der Kolonne anreichern. Mittelsieder in diesem Zusammenhang ist insbesondere Dodecan-1-ol. Zur Fraktion der Mittelsieder gesellen sich weitere organische Substanzen, die bisher nicht genau charakterisiert werden konnten.

"Schwersieder" im Sinne dieser Erfindung sind Stoffe oder Stoffgemische, die bei denselben Druckbedingungen einen höheren Siedepunkt als CDOL aufweisen und daher bei der destillativen Trennung eines Gemisches aus Schwersiedern und CDOL im Rückstand verbleiben. Die Schwersiedergrenze liegt etwa bei 180°C bei einem Druck von 46 mbar. Zu den Schwersiedern gehört insbesondere Cyclododecandiol. Darüber hinaus enthält die Fraktion der Schwersieder weitere organische Substanzen, die bisher nicht näher charakterisiert werden konnten.

Die Dehydrierung von CDOL zu CDON ist beschrieben in den Dokumenten DE1568317 sowie in DE1248650. Dabei werden Dehydrierungsgemische beschrieben, die 74 bis 89 Gew.-% CDON und 25.9 bis 21.8 Gew.-% CDOL enthalten. Der Restanteil des hergestellten Dehydrierungsgemisches fällt auf Leichtsieder und Mittelsieder. Die Aufarbeitung des Dehydrierungsgemisches ist nicht weiter beschrieben.

Aus der japanischen Patentanmeldung JP05-000977A ist ein Verfahren zur Herstellung von hochreinem CDOL aus einem CDON/CDOL-Gemisch bekannt. Während der destillativen Aufarbeitung des Gemisches wird ein geringer Anteil an alkalischen Komponenten dem aufzutrennenden Gemisch zugegeben.

Die Oxidation von CDAN in ein Oxidationsgemisch enthaltend CDAN und CDOL ist in GB930842 beschrieben. Die nachfolgenden Verarbeitungsschritte sind nicht dargestellt.

In der DE2031782 ist ein Verfahren zur selektiven Herstellung von CDON beschrieben, bei dem CDAN oxidiert wird, um ein Gemisch aus CDON und CDOL zu erhalten. Das Gemisch wird destillativ aufgearbeitet, jedoch ohne nähere Beschreibung des Destillationsprozesses.

DE 102005048250 A1 beschreibt ein Verfahren zur Reinigung einer Zusammensetzung enthaltend CDON. Das Verfahren umfasst unter anderem die destillative Reinigung.

Das für die Herstellung von Laurinlactam verwendete CDON sollte in einer möglichst reinen Form vorliegen, da Begleitkomponenten die Polymere im Polyamid 12 nachhaltig schädigen. Diese Nebenkomponenten entstehen insbesondere während der Oxidation des CDAN und auch während der Dehydrierung des CDOL.

Im Hinblick auf diesen Stand der Technik ist es Aufgabe der Erfindung, ein Verfahren zur Aufarbeitung eines Gemisches enthaltend Leichtsieder, CDON, Mittelsieder, CDOL und Schwersieder anzugeben, bei dem eine Ziel-Fraktion erhalten wird, die aus möglichst reinem CDON besteht.

Gelöst wird diese Aufgabe durch ein Verfahren mit den Merkmalen des Anspruch 1.

Gegenstand der Erfindung ist mithin ein Verfahren zur Abtrennung einer Cyclododecanon-reichen Ziel-Fraktion aus einem Leichtsieder, Cyclododecanon, Mittelsieder, Cyclododecanol und Schwersieder enthaltenden Dehydrierungsgemisch, welches die folgenden Schritte aufweist:
a) Bereitstellen des Dehydrierungsgemisches;
b) destillatives Abtrennen der Leichtsieder aus dem Dehydrierungsgemisch unter Erhalt eines ersten Gemisches umfassend Cyclododecanon, Mittelsieder, Cyclododecanol und Schwersieder;
c) Einspeisen des ersten Gemisches in eine primäre Seitenabzugskolonne;
d) Abziehen einer ersten Cyclododecanon-reichen Fraktion vom Kopf der primären Seitenabzugskolonne;
e) Abziehen einer ersten Cyclododecanol und Schwersieder enthaltenden Fraktion vom Sumpf der primären Seitenabzugskolonne;
f) Abziehen eines zweiten Gemisches umfassend Cyclododecanon, Cyclododecanol und Mittelsieder aus dem Seitenabzug der primären Seitenabzugskolonne;
g) Einspeisen des zweiten Gemisches in eine sekundäre Seitenabzugskolonne;
h) Abziehen einer zweiten Cyclododecanon-reichen Fraktion vom Kopf der sekundären Seitenabzugskolonne;
i) Abziehen einer zweiten Cyclododecanol und Schwersieder enthaltenden Fraktion vom Sumpf der sekundären Seitenabzugskolonne;
j) Abziehen eines dritten Gemisches umfassend Cyclododecanon, Cyclododecanol und Mittelsieder aus dem Seitenabzug der sekundären Seitenabzugskolonne;
k) Vereinigen der ersten Cyclododecanon-reichen Fraktion und der zweiten Cyclododecanon-reichen Fraktion unter Erhalt der Cyclododecanon-reichen Ziel-Fraktion.

Grundidee der vorliegenden Erfindung ist die Verwendung einer Sequenz zweier hintereinander geschalteter Seitenabzugskolonnen, wobei der Seitenstrom der primären Seitenabzugskolonne in die sekundäre Seitenabzugskolonne eingespeist wird. Vom Kopf beider Seitenabzugskolonnen wird jeweils eine CDON-reiche Fraktion abgezogen, welche zu einer Ziel-Fraktion vereinigt werden, die im Wesentlichen reines CDON darstellt. Der störende Mittelsieder wird über die Seite abgezogen. Über den Sumpf werden CDOL und die Schwersieder abgeschieden.

In einer bevorzugten Ausführungsform der Erfindung wird das zweite Gemisch flüssig aus der primären Seitenabzugkolonne abgezogen und flüssig in die sekundäre Seitenabzugskolonne eingebracht.

Um sicher zu stellen, dass ausschließlich flüssige Stoffe den Seitenabzug der primären Seitenabzugskolonne verlassen, sollte der Seiteabzug an einem Flüssigkeitssammler in der primären Seitenabzugskolonne angeordnet sein. Flüssigkeitssammler sind in der Destillationstechnik gemeinhin bekannt und dienen in erster Linie dazu, die von einem Packungs- oder Schüttungsbett herabrieselnde Flüssigkeit zu sammeln und auf einen Flüssigkeitsverteiler zu geben, der die Flüssigkeit auf ein darunter liegendes Bett gleichmäßig verteilt. In der sekundären Seitenabzugskolonne wird das zweite Gemisch wie ein Feed flüssig aufgegeben. Mithin wird bevorzugt ein ausschließlich flüssiger Stoffstrom zwischen den beiden Kolonnenteilen ausgetauscht.

In einer ganz besonders bevorzugten Ausführungsform der Erfindung wird die Verbindung zwischen den beiden Seitenabzugskolonnen so positioniert, dass das zweite Gemisch auf der Trennebene der primären Seitenabzugskolonne abgezogen wird, auf welcher die flüssige Konzentration der Mittelsieder in der primären Seitenkolonne maximal ist, und dass das zweite Gemisch auf eine Trennebene der sekundären Seitenabzugskolonne eingespeist wird, auf welcher die Zusammensetzung des zweiten Gemisches im Wesentlichen der Zusammensetzung der flüssigen Phase auf dieser Trennebene der primären Seitenabzugskolonne entspricht.

Dies bedeutet, dass in der primären Seitenabzugskolonne der Mittelsieder auf wenige Prozente aufkonzentriert wird, am Konzentrationsbauch angezapft und dieser Strom in die sekundäre Kolonne wieder eingespeist wird. Die sekundäre Kolonne kann dann mit einem erhöhten Rücklaufverhältnis betrieben werden, sodass der Mittelsieder auf hohe Werte von bis zu 40 % aufkonzentriert und im sekundären Seitenstrom entnommen werden kann.

Die beiden Cyclododecanol und Schwersieder enthaltenden Sumpf-Fraktionen der primären und sekundären Seitenabzugskolonne werden bevorzugt vereinigt und einer die Schwersieder zumindest teilweise abtrennenden Destillationsstufe zugeführt. Bei dieser Destillationsstufe handelt es sich vorzugsweise um eine zwischen CDAN-Oxidation und CDOL-Dehydrierung angeordnete Vorabscheiderkolonne.

Vorzugsweise werden beide Seitenabzugskolonnen unter Vakuum gefahren; also bei einem Druck unter 1 bar absolut. Insbesondere sollte der Druck in beiden Seitenabzugskolonnen unter 50 mbar absolut liegen.

Es bietet sich an, den Unterdruck in den Seitenabzugskolonnen mit einem gemeinsamen Vakuumaggregat zu erzeugen.

Vorzugsweise dient das erfindungsgemäße Verfahren dazu, ein Dehydrierungsgemisch aufzuarbeiten, welches die folgende, sich zu 100 % ergänzende Zusammensetzung aufweist:
Leichtsieder (LB): 1 bis 8 Gew-%, bevorzugt 3 Gew-%;
Cyclododecanon (CDON): 60 bis 90 Gew-%, bevorzugt 70 Gew-%;
Mittelsieder (MB): 0 bis 1.5 Gew-%, bevorzugt 1 Gew-%;
Cyclododecanol (CDOL): 10 bis 40 Gew-%, bevorzugt 24 Gew-%;
Schwersieder (HB): 0.1 bis 2.5 Gew-%, bevorzugt 2 Gew-%.

Das Verfahren dient vorzugsweise dazu, eine Ziel-Fraktion zu erhalten, die einen besonders hohen Reinheitsgrad aufweist. Erfindungsgemäß sollte die Ziel-Fraktion einen CDON-Gehalt von mindestens 98 Gew.-% aufweisen, bevorzugt wird sogar ein CDON-Gehalt von 99,5 Gew.-% angestrebt. Des Weiteren sollten die am Kopf der Seitenabzugskolonnen abgezogenen, Cyclododecanon-reichen Fraktionen möglichst frei von CDOL, Schwersiedern und Mittelsiedern sein. Die am Sumpf der Seitenabzugskolonnen abgezogenen Fraktionen sollten demgegenüber möglichst frei von CDON sein.

Sofern das erfindungsgemäße Verfahren im Zuge eines Laurinlactam-Prozesses eingesetzt wird, umfasst der Arbeitsschritt "Bereitstellen des Dehydrierungsgemisches" die folgenden Teilschritte:
I) Oxidation von Cyclododecan mit Sauerstoff unter Erhalt eines Oxdiationsgemisches enthaltend Leichtsieder, Cyclododecanon, Mittelsieder, Cyclododecanol und Hochsieder;
m) destillatives Abtrennen einer Cyclododecanol-reichen Fraktion aus dem Oxidationsgemisch, welches hinsichtlich Schwersieder abgereichert ist;
n) Dehydrierung der Cyclododecanol-reichen Fraktion unter Erhalt des Dehydrierungsgemisches.

Sofern das erfindungsgemäße Verfahren Teil eines Laurinlactam-Prozesses ist, empfiehlt es sich, den Sumpf der beiden Seitenabzugskolonnen in den in einer Vorabscheiderkolonne durchgeführten Schritt m) zurückzuführen. Die Sumpffraktionen enthalten nämlich zu einem großen Teil CDOL, das auf diese Weise dem Dehydrierungsprozess wieder zugängig gemacht und in CDON umgesetzt werden kann. Die Schwersieder konzentrieren sich bis zu einer Grenzkonzentration auf und werden mit dieser im Kreis geführt. Die über die Oxidation in den Prozess neu eingetragenen Schwersieder verlassen den Prozess wieder über den Sumpf der Vorabscheiderkolonne und über den sekundären Seitenabzug.

Das vereinigte Kopfprodukt der Seitenabzugskolonnen stellt hochreines CDON dar, welches sich hervorragend dazu eignet, oximiert und sodann zu Laurinlactam weiter verarbeitet zu werden. Das über den sekundären Seitenabzug gelieferte Gemisch kann entweder stofflich oder thermisch verwertet werden. Bevorzugt wird es stofflich verwertet, indem das Gemisch gesammelt und mittels einer Batch-Destillation aufgetrennt wird. Ziel dabei ist die verbleibenden Anteile CDON zu gewinnen. Falls dies wirtschaftlich nicht vertretbaren Aufwand bedeutet, kann das über den sekundären Seitenabzug gelieferte dritte Gemisch auch verbrannt werden. Die fühlbare Wärme kann ggf. vorher noch abgegriffen werden.

Weitere vorteilhafte Merkmale der Erfindung gehen aus der Beschreibung und den Beispielen hervor.

Die Erfindung soll nun anhand von Ausführungsbeispielen näher erläutert werden. Hierfür zeigen:
- Figur 1:: Verfahrensfließbild;
- Figur 2:: Detail Seitenabzugskolonnen mit Konzentrationsprofilen.

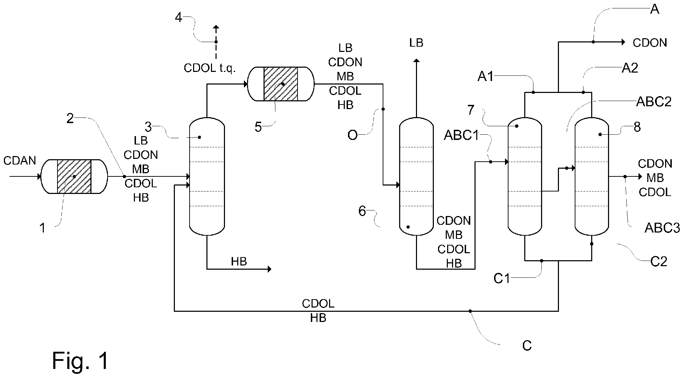

Der gesamte Aufarbeitungsprozess ist in Figur 1 dargestellt. Er beginnt in einer Oxidation 1, in welcher CDAN mit Sauerstoff oxidiert wird. Dabei fällt ein Oxidationsgemisch 2 an, welches Leichtsieder LB, CDON, Mittelsieder MB, CDOL und Hochsieder HB enthält. Aufgrund des Reaktionsmechanismus ist der Gehalt an CDON innerhalb des Oxidationsgemisches 2 deutlich geringer als der des CDOL. Typischerweise enthält ein derartiges Oxidationsgemisch 2 etwa 15 Gew.-% CDON und etwa 70 Gew.-% CDOL.

Das Oxidationsgemisch 2 wird in eine Vorabscheiderkolonne 3 eingespeist. Die Funktion dieser Vorabscheiderkolonne 3 besteht darin, einen großen Anteil der Hochsieder HB auszuschleusen. Dies geschieht über den Sumpf. Über den Kopf der Vorabscheiderkolonne 3 wird CDOL t.q. abgezogen. CDOL t.q. enthält etwa 84 Gew.-% CDOL und 13 Gew.-% CDON. CDOL t.q. stellt ein separates Verkaufsprodukt dar und kann gegebenenfalls über einen Abzweig 4 aus dem Prozess ausgeschleust werden.

Zur Laurinlactamherstellung wird CDOL t.q. einer Dehydrierung 5 unterworfen. Dabei wird CDOL zu CDON dehydriert, sodass sich der Anteil dieser beiden Stoffe umkehrt. Das aus der Dehydrierung 5 abgezogene Dehydrierungsgemisch O weist typischerweise die folgende, sich zu 100% ergänzende Zusammensetzung auf:

| | |
|---|---|
| Leichtsieder (LB): | 1 bis 8 Gew-%, bevorzugt 3 Gew-%; |
| Cyclododecanon (CDON): | 60 bis 90 Gew-%, bevorzugt 70 Gew-%; |
| Mittelsieder (MB): | 0 bis 1.5 Gew-%, bevorzugt 1 Gew-%; |

| | |
|---|---|
| Cyclododecanol (CDOL): | 10 bis 40 Gew-%, bevorzugt 24 Gew-%; |
| Schwersieder (HB): | 0.1 bis 2.5 Gew-%, bevorzugt 2 Gew-%. |

Das Dehydrierungsgemisch O wird nun in eine Leichtsiederkolonne 6 eingespeist. Zweck der Leichtsiederkolonne 6 ist es, die Leichtsieder LB über Kopf destillativ aus dem Dehydrierungsgemisch O abzutrennen, sodass am Sumpf der Leichtsiederkolonne 6 ein erstes Gemisch ABC1 umfassend CDON, Mittelsieder MB, CDOL und Schwersieder HB anfällt. Die Leichtsieder LB werden in diesem Schritt vorzugsweise vollständig abgetrennt. Die Besonderheit des Sumpfproduktes ABC1 besteht darin, dass sein Gehalt an Mittelsiedern MB äußerst gering ist, nämlich lediglich etwa 1 Gew.-% beträgt. Im Übrigen besteht das Gemisch ABC1 im Wesentlichen aus CDON, CDOL und Hochsiedern HB. Eine typische, sich zu 100% ergänzende Zusammensetzung des Gemisches ABC1 ist wie folgt:

| | |
|---|---|
| Leichtsieder (LB): | 0 bis 1 Gew-%, bevorzugt 0 Gew-%; |
| Cyclododecanon (CDON): | 60 bis 90 Gew-%, bevorzugt 70 Gew-%; |
| Mittelsieder (MB): | 0 bis 2 Gew-%, bevorzugt 1 Gew-%; |
| Cyclododecanol (CDOL): | 10 bis 40 Gew-%, bevorzugt 26 Gew-%; |
| Schwersieder (HB): | 0.1 bis 3 Gew-%, bevorzugt 3 Gew-%. |

Gemisch ABC1 wird sodann in eine primäre Seitenabzugskolonne 7 eingespeist. Vom Kopf der primären Seitenabzugskolonne 7 wird ein CDON-reiche Fraktion A1 abgezogen. Vorzugsweise beträgt der Anteil an CDON in Fraktion A1 mindestens 95 Gew.-%. Ideal ist das Kopfprodukt A1 frei von CDOL, Mittelsiedern MB und Schwersiedern HB, was sich in der Praxis nicht mit wirtschaftlich vertretbarem Aufwand realisieren lässt. Eine Reinheit von > 99 Gew.-% lässt sich aber erzielen.

Eine typische, sich zu 100% ergänzende Zusammensetzung des Gemisches A1 ist wie folgt:

| | |
|---|---|
| Leichtsieder (LB): | 0 bis 1 Gew-%, bevorzugt 0 Gew-%; |
| Cyclododecanon (CDON): | 95 bis 100 Gew-%, bevorzugt 99.5 Gew-%; |
| Mittelsieder (MB): | 0 bis 1 Gew-%, bevorzugt 0.25 Gew-%; |
| Cyclododecanol (CDOL): | 0 bis 1 Gew-%, bevorzugt 0.25 Gew-%; |
| Schwersieder (HB): | 0 bis 0.5 Gew-%, bevorzugt 0 Gew-%. |

Am Sumpf der primären Seitenabzugskolonne 7 wird eine erste CDOL und Schwersieder HB enthaltenden Fraktion C1 abgezogen. Ideal ist diese frei von Mittelsiedern MB und CDON.

Eine typische, sich zu 100% ergänzende Zusammensetzung des Gemisches C1 ist wie folgt:

| | |
|---|---|
| Leichtsieder (LB): | 0 bis 1 Gew-%, bevorzugt 0 Gew-%; |
| Cyclododecanon (CDON): | 0 bis 1 Gew-%, bevorzugt 0 Gew-%; |
| Mittelsieder (MB): | 0 bis 1 Gew-%, bevorzugt 0.2 Gew-%; |
| Cyclododecanol (CDOL): | 85 bis 95 Gew-%, bevorzugt 90 Gew-%; |
| Schwersieder (HB): | 5 bis 15 Gew-%, bevorzugt 9.8 Gew-%. |

Von dem Seitenabzug der primären Seitenabzugskolonne 7 wird ein zweites Gemisch ABC2 abgezogen. ABC2 weist die folgende, sich zu 100 % ergänzende Zusammensetzung auf:

| | |
|---|---|
| Cyclododecanon (CDON): | 30 bis 50 Gew-%, bevorzugt 43 Gew-%; |
| Mittelsieder (MB): | 0 bis 20 Gew-%, bevorzugt 18 Gew-%; |
| Cyclododecanol (CDOL): | 30 bis 50 Gew-%, bevorzugt 38.4 Gew-%; |
| Schwersieder (HB): | 0.1 bis 2 Gew-%, bevorzugt 0.6 Gew-%. |

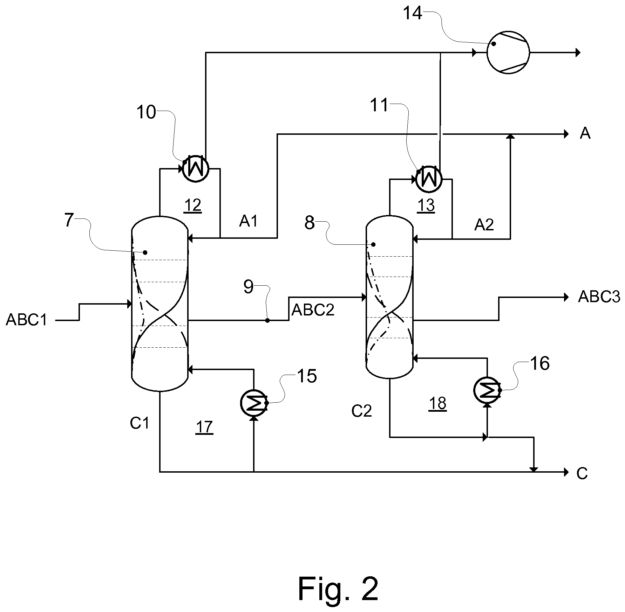

Gemisch ABC2 wird flüssig aus der primären Seitenabzugskolonne 7 abgezogen. Zu diesem Zwecke ist der Seitenabzug an einem Flüssigkeitssammler angeordnet (nicht dargestellt). Der Seitenabzug befindet sich auf der Trennebene, auf welcher die Konzentration der Mittelsieder in der primären Seitenabzugskolonne 7 maximal ist. Einzelheiten dazu werden noch anhand der Figur 2 erläutert werden.

Das zweite Gemisch ABC2 wird flüssig in eine sekundäre Seitenabzugskolonne 8 eingespeist. Dies erfolgt auf der Trennebene, an welcher die Zusammensetzung der flüssigen Phase innerhalb der sekundären Seitenabzugskolonne 8 im Wesentlichen die des Gemisches ABC2 entspricht. Einzelheiten dazu werden noch anhand der Figur 2 erläutert werden.

Vom Kopf der sekundären Seitenabzugskolonne 8 wird eine zweite CDON-reiche Fraktion A2 abgezogen. Vorzugsweise beträgt der Anteil an CDON in Fraktion A2 mindestens 97 Gew.-%. Ideal ist das Kopfprodukt A2 frei von CDOL, Mittelsiedern MB und Schwersiedern HB, was sich in der Praxis nicht mit wirtschaftlich vertretbarem Aufwand realisieren lässt. Eine Reinheit von > 99 Gew.-% lässt sich aber erzielen.

Eine typische, sich zu 100% ergänzende Zusammensetzung des Gemisches A2 ist wie folgt:

| | |
|---|---|
| Leichtsieder (LB): | 0 bis 1 Gew-%, bevorzugt 0 Gew-%; |
| Cyclododecanon (CDON): | 97 bis 100 Gew-%, bevorzugt 99.9 Gew-%; |
| Mittelsieder (MB): | 0 bis 1 Gew-%, bevorzugt 0.05 Gew-%; |
| Cyclododecanol (CDOL): | 0 bis 1 Gew-%, bevorzugt 0.05 Gew-%; |
| Schwersieder (HB): | 0 bis 0.5 Gew-%, bevorzugt 0 Gew-%. |

Am Sumpf der sekundären Seitenabzugskolonne 8 wird eine zweite CDOL und Schwersieder HB enthaltenden Fraktion C2 abgezogen. Ideal ist diese frei von Mittelsiedern MB und CDON.

Eine typische, sich zu 100% ergänzende Zusammensetzung des Gemisches C2 ist wie folgt:

| | |
|---|---|
| Leichtsieder (LB): | 0 bis 1 Gew-%, bevorzugt 0 Gew-%; |
| Cyclododecanon (CDON): | 0 bis 1 Gew-%, bevorzugt 0 Gew-%; |
| Mittelsieder (MB): | 0 bis 1 Gew-%, bevorzugt 0.2 Gew-%; |
| Cyclododecanol (CDOL): | 85 bis 95 Gew-%, bevorzugt 90 Gew-%; |
| Schwersieder (HB): | 5 bis 15 Gew-%, bevorzugt 9.8 Gew-%. |

Über den Seitenabzug der sekundären Seitenabzugskolonne 8 wird ein an Mittelsiedern reiches, drittes Gemisch ABC3 abgezogen. Es enthält im Wesentlichen CDON, CDOL und Mittelsieder. Die Mittelsieder MB stellen dabei den größten Anteil des Seitengemisches ABC3 dar. Der Anteil der Wertprodukte CDON und CDOL ist geringer. Der über den Seitenabzug der sekundären Seitenabzugskolonne 8 entnommene Stoffstrom ABC3 weist typischerweise die folgende, sich zu 100 % ergänzende Zusammensetzung auf:

| | |
|---|---|
| Cyclododecanon (CDON): | 20 bis 50 Gew-%, bevorzugt 35 Gew-%; |
| Mittelsieder (MB): | 0 bis 60 Gew-%, bevorzugt Gew-%; |
| Cyclododecanol (CDOL): | 10 bis 50 Gew-%, bevorzugt Gew-%; |
| Schwersieder (HB): | 0 bis 2 Gew-%, bevorzugt 1 Gew-%. |

Das über den Seitenabzug der sekundären Seitenabzugskolonne 8 abgezogene Gemisch ABC3 kann entweder stofflich oder thermisch verwertet werden. Bevorzugt ist eine stoffliche Verwertung die darin besteht, ABC3 zu sammeln und einer Batch-Destillation zu unterziehen. Auf diese Weise wird aus ABC3 das wertvolle CDON gewonnen. Alternativ kann ABC3 verbrannt werden. Nach Möglichkeit sollte zuvor die von ABC3 getragene, fühlbare Wärme abgegriffen werden.

Die beiden Kopfprodukte A1 und A2 werden vereint zu einer Fraktion A, die nahezu reines CDON darstellt. Dieses für die Laurinlactam-Herstellung geeignete, hochreine CDON Gemisch stellt die Ziel-Fraktion des Prozesses dar. Ziel-Fraktion A besteht zu mindestens 98 Gew.-% aus CDON, bevorzugt sogar zu 99.5 Gew.-% aus CDON.

Die beiden Sumpfprodukte C1 und C2 werden vereint zu einer Fraktion C, die im Wesentlichen CDOL und Schwersieder HB enthält. Die vereinte Sumpffraktion C wird recycliert und zusammen mit dem Oxidationsgemisch 2 in die Vorabscheiderkolonne 3 geführt. Auf diese Weise wird das nicht umgesetzte CDOL wieder der Dehydrierung 5 zugeführt. Die zurückgeführten Hochsieder HB konzentrieren sich in dem Prozess zu einem bestimmten Maße auf. Die aus der Oxidation 1 frisch herein getragenen Hochsieder HB werden über den Sumpf der Vorabscheiderkolonne 3 ausgeschieden, sodass sich hinsichtlich der Hochsieder-Konzentration ein stationärer Zustand einstellt.

Der schematische Aufbau der beiden hintereinander geschalteten Seitenabzugskolonnen 7, 8 ist in Figur 2 detaillierter dargestellt.

Zunächst ist zu erwähnen, dass in beide Kolonnen 7, 8 eine Vielzahl von an sich bekannten Einbauten eingebaut sein können, wie beispielsweise Packungen oder Füllkörper der Firma Sulzer oder der Firma Montz. Zweck der Einbauten ist es, eine möglichst große Anzahl von theoretischen Trennstufen zu erreichen. Vorzugsweise verfügen die beiden Kolonnen 7, 8 über die folgende Anzahl von theoretischen Trennstufen:

| | |
|---|---|
| Primäre Seitenabzugskolonne 7: | 75 theoretische Trennstufen |
| Sekundäre Seitenabzugskolonne 8: | 75 theoretische Trennstufen |

Des Weiteren können in beiden Kolonnen 7, 8 verschiedene Flüssigkeitssammler und -verteiler vorgesehen sein, baulich ausgeführt in fachüblicher Weise.

Wesentliches Merkmal der erfindungsgemäßen Sequenz der beiden Seitenabzugskolonnen 7,8 ist die Verbindung 9, welche den Seitenabzug der primären Seitenabzugskolonne 7 mit dem Einlauf der sekundären Seitenabzugskolonne 8 verbindet. Bei der Verbindung 9 handelt es sich um eine Flüssigkeitsleitung, die zur Herstellung einer Strömung durch die Leitung mit einer nicht dargestellten Zirkulationspumpe versehen ist. Mittels der Verbindung 9 wird flüssiger Mittelsieder von dessen Konzentrationsmaximum in der primären Seitenabzugskolonne 7 zur sekundären Seitenabzugskolonne 8 transferiert. Um sicher zu stellen, dass über die Verbindung 9 ausschließlich flüssige Stoffe ausgetauscht werden, entspringt die Verbindung 9 von einem Flüssigkeitssammler in der primären Seitenabzugskolonne 7. Der Flüssigkeitssammler ist auf einer Trennebene eingebaut, an welcher die flüssige Konzentration der Mittelsieder ein Maximum aufweist. Die Verbindung 9 entspringt damit auf der Trennebene der primären Seitenabzugskolonne 7, auf welcher die flüssige Konzentration im Mittelsieder in dieser Kolonne 7 maximal ist. Die Verbindung 9 mündet auf der Trennebene der sekundären Seitenabzugskolonne 7, auf welcher die Zusammensetzung der flüssigen Phase möglichst derjenigen des der primären Seitenabzugskolonne 7 entnommenen, flüssigen Gemisches ABC2 entspricht. Dies bedeutet, dass in der primären Seitenabzugskolonne 7 der Mittelsieder auf wenige Prozente aufkonzentriert wird, am Konzentrationsbauch angezapft und dieser Strom ABC2 über die Verbindung 9 der sekundären Seitenabzugskolonne 8 aufgegeben wird. Abzugs- und Einspeisepunkt der Verbindung 9 werden demnach den Konzentrationsverhältnissen entsprechend gewählt und müssen nicht auf derselben Ebene liegen.

Bei der oben genannten Anzahl von theoretischen Trennstufen sind die Einspeise- und Abzugspunkte der Gemische ABC1, ABC2 und ABC3 vorzugsweise auf den folgenden Trennstufen der entsprechenden Kolonne angeordnet (von oben gezählt):

| | ABC 1 | ABC 2 | ABC 3 |
|---|---|---|---|
| Einspeisung: | 35 | 60 | - |
| Abzug: | - | 65 | 55 |

Von den Köpfen der beiden Seitenabzugskolonnen 7, 8 wird jeweils ein gasförmiges Kopfprodukt A1, A2 abgezogen, welches fast ausschließlich CDON enthält. Wie bei Destillationskolonnen üblich, sind am Kopfabzug jeweils ein Kondensator 10, 11 und ein Kopfrücklauf 12, 13 vorgesehen. Ein gemeinsames Vakuumsystem 14 baut in beiden Seitenabzugskolonnen 7, 8 einen Unterdruck von etwa 40 mbar absolut auf.

Das Rücklaufverhältnis v wird unter den geschilderten Bedingungen wie folgt gewählt:

| | |
|---|---|
| Primäre Seitenabzugskolonne 7: | v= 4.3 |
| Sekundäre Seitenabzugskolonne 8: | v= 3.5 |

An den Sümpfen der beiden Seitenabzugskolonnen 7, 8 wird jeweils eine flüssige Fraktion C1, C2 abgezogen, die überwiegend CDOL sowie Hochsieder enthält. Wie bei Destillationskolonnen üblich, ist hier ein Sumpferhitzer/Verdampfer 15, 16 mit dem entsprechenden Sumpfrücklauf 17, 18 vorgesehen.

In Figur 2 sind auch die Konzentrationsprofile von CDON (durchgezogene Linie, Fraktion A), CDOL (gestrichelte Linie, Fraktion C) und Mittelsieder (strichpunktierte Linie, Fraktion B) eingezeichnet. Die Verbindung 9 ist so angeordnet, dass sie auf der Trennebene der primären Kolonne 7 entspringt, auf welcher die flüssige Konzentration im Mittelsieder maximal ist. Die Verbindung mündet auf der Trennebene der sekundären Seitenabzugskolonne 8, auf welcher die Zusammensetzung der flüssigen Phase möglichst derjenigen des von der primären Kolonne entnommenen, flüssigen Stroms ABC2 entspricht. Dies bedeutet, dass in der primären Kolonne der Mittelsieder auf wenige Prozente aufkonzentriert wird, am Konzentrationsbauch angezapft und dieser Strom über die Verbindung 9 in die sekundäre Kolonne wieder eingespeist wird. Auf diese Weise wird in der primären Kolonne das Gemisch ABC1 so getrennt, dass CDON als reines Destillat und CDOL als reiner Schwersieder gewonnen werden. Der Mittelsieder bildet innerhalb der primären Kolonne einen Konzentrationsbauch aus. An der Stelle des Konzentrationsmaximums wird ein Teilstrom flüssigen Mittelsieders entnommen und über die Verbindung 9 in die sekundäre Kolonne überführt. Dort wird mit erhöhtem Rücklauf erneut eine scharfe Trennung von CDON und CDOL durchgeführt. Dabei wird der Mittelsieder wiederum innerhalb der sekundären Kolonne stark aufkonzentriert und kann über den Seitenstrom ABC3 abgeführt werden.

### Bezugszeichenliste

- 1: Oxidation
- 2: Oxidationsgemisch
- 3: Vorabscheiderkolonne
- 4: Abzweig
- 5: Dehydrierung
- 6: Leichtsiederkolonne
- 7: primäre Seitenabzugskolonne
- 8: sekundäre Seitenabzugskolonne
- 9: Verbindung
- 10: Kondensator der primären Seitenabzugskolonne
- 11: Kondensator der sekundären Seitenabzugskolonne
- 12: Kopfrücklauf der primären Seitenabzugskolonne
- 13: Kopfrücklauf der sekundären Seitenabzugskolonne
- 14: Vakuumsystem
- 15: Sumpferhitzer / Verdampfer der primären Seitenabzugskolonne
- 16: Sumpferhitzer / Verdampfer der sekundären Seitenabzugskolonne
- 17: Sumpfrücklauf der primären Seitenabzugskolonne
- 18: Sumpfrücklauf der sekundären Seitenabzugskolonne
- O: Dehydrierungsgemisch
- A: Ziel-Fraktion
- A1: Kopfprodukt der primären Seitenabzugskolonne
- A2: Kopfprodukt der sekundären Seitenabzugskolonne
- C: CDON/Hochsieder-haltige Fraktion
- C1: Sumpfprodukt der primären Seitenabzugskolonne
- C2: Sumpfprodukt der sekundären Seitenabzugskolonne
- ABC1: erstes Gemisch (Feed primäre Seitenabzugskolonne)
- ABC2: zweites Gemisch (primärer Seitenabzug)
- ABC3: drittes Gemisch (sekundärer Seitenabzug)

## Patentansprüche

1. Verfahren zur Abtrennung einer Cyclododecanon-reichen Ziel-Fraktion (A) aus einem Leichtsieder (LB), Cyclododecanon (CDON), Mittelsieder (MB), Cyclododecanol (CDOL) und Schwersieder (HB) enthaltenden Dehydrierungsgemisch (O), **gekennzeichnet durch** die folgenden Schritte:
a) Bereitstellen des Dehydrierungsgemisches (O);
b) destillatives Abtrennen der Leichtsieder (LB) aus dem Dehydrierungsgemisch (O) unter Erhalt eines ersten Gemisches (ABC1) umfassend Cyclododecanon (CDON), Mittelsieder (MB), Cyclododecanol (CDOL) und Schwersieder (HB);
c) Einspeisen des ersten Gemisches (ABC1) in eine primäre Seitenabzugskolonne (7);
d) Abziehen einer ersten Cyclododecanon-reichen Fraktion (A1) vom Kopf der primären Seitenabzugskolonne (7);
e) Abziehen einer ersten Cyclododecanol (CDOL) und Schwersieder (HB) enthaltenden Fraktion (C1) vom Sumpf der primären Seitenabzugskolonne (7);
f) Abziehen eines zweiten Gemisches (ABC2) umfassend Cyclododecanon (CDON), Cyclododecanol (CDOL) und Mittelsieder (MB) aus dem Seitenabzug der primären Seitenabzugskolonne (7);
g) Einspeisen des zweiten Gemisches (ABC2) in eine sekundäre Seitenabzugskolonne (8);
h) Abziehen einer zweiten Cyclododecanon-reichen Fraktion (A2) vom Kopf der sekundären Seitenabzugskolonne (8);
i) Abziehen einer zweiten Cyclododecanol (CDOL) und Schwersieder (HB) enthaltenden Fraktion (C2) vom Sumpf der sekundären Seitenabzugskolonne (8);
j) Abziehen eines dritten Gemisches (ABC3) umfassend Cyclododecanon (CDON), Cyclododecanol (CDOL) und Mittelsieder (MB) aus dem Seitenabzug der sekundären Seitenabzugskolonne (8);
k) Vereinigen der ersten Cyclododecanon-reichen Fraktion (A1) und der zweiten Cyclododecanon-reichen Fraktion (A2) unter Erhalt der Cyclododecanon-reichen Ziel-Fraktion (A).

2. Verfahren nach Anspruch 1, **dadurch gekennzeichnet, dass** das zweite Gemisch (ABC2) flüssig aus der primären Seitenabzugskolonne abgezogen und flüssig in die sekundäre Seitenabzugskolonne eingebracht wird.

3. Verfahren nach Anspruch 2, **dadurch gekennzeichnet, dass** das zweite Gemisch (ABC2) auf der Trennebene der primären Seitenabzugskolonne abgezogen wird, auf welcher die flüssige Konzentration der Mittelsieder (MB) in der primären Seitenkolonne maximal ist, und dass das zweite Gemisch (ABC2) auf eine Trennebene der sekundären Seitenabzugskolonne eingespeist wird, auf welcher die Zusammensetzung des zweiten Gemisches (ABC2) im Wesentlichen der Zusammensetzung der flüssigen Phase auf dieser Trennebene der primären Seitenabzugskolonne entspricht.

4. Verfahren nach einem der Ansprüche 1 bis 3, **dadurch gekennzeichnet**, die erste Cyclododecanol (CDOL) und Schwersieder (HB) enthaltenden Fraktion (C1) und die zweite Cyclododecanol (CDOL) und Schwersieder (HB) enthaltenden Fraktion (C2) vereinigt einer die Schwersieder zumindest teilweise abtrennenden Destillationsstufe zugeführt werden.

5. Verfahren nach einem der Ansprüche 1 bis 4, **dadurch gekennzeichnet, dass** beide Seitenabzugskolonnen bei einem Druck von unter 1 bar absolut, insbesondere unter 50 mbar absolut betrieben werden.

6. Verfahren nach Anspruch 5, **dadurch gekennzeichnet, dass** der Unterdruck in den Seitenabzugskolonnen von einem gemeinsamen Vakuumaggregat erzeugt wird.

7. Verfahren nach einem der Ansprüche 1 bis 6, **dadurch gekennzeichnet, dass** das Dehydrierungsgemisch (O) die folgende, sich zu 100 % ergänzende Zusammensetzung aufweist:
Leichtsieder (LB): 1 bis 8 Gew-%, bevorzugt 3 Gew-%;
Cyclododecanon (CDON): 60 bis 90 Gew-%, bevorzugt 70 Gew-%;
Mittelsieder (MB): 0 bis 1.5 Gew-%, bevorzugt 1 Gew-%;
Cyclododecanol (CDOL): 10 bis 40 Gew-%, bevorzugt 24 Gew-%;
Schwersieder (HB): 0.1 bis 2.5 Gew-%, bevorzugt 2 Gew-%.

8. Verfahren nach einem der Ansprüche 1 bis 7, **dadurch gekennzeichnet, dass** die Cyclododecanon-reiche Ziel-Fraktion (A) einen Gehalt an Cyclododecanon (CDON) von mindestens 98 Gew.-%, bevorzugt von 99.5 Gew.-% aufweist, und/oder dass die Cyclododecanon-reiche Ziel-Fraktion (A) frei von Cyclododecanol (CDOL), Schwersiedern (HB) und Mittelsiedern (MB) ist.

9. Verfahren nach einem der Ansprüche 1 bis 8, **dadurch gekennzeichnet, dass** die Cyclododecanol (CDOL) und Schwersieder (HB) enthaltende Fraktionen (C1, C2) jeweils frei von Cyclododecanon (CDON) sind.

10. Verfahren nach einem der Ansprüche 1 bis 9, **dadurch gekennzeichnet, dass** das Bereitstellen des Dehydrierungsgemisches (O) die folgenden Schritte umfasst:
I) Oxidation von Cyclododecan (CDAN) mit Sauerstoff unter Erhalt eines Oxdiationsgemisches (2) enthaltend Leichtsieder (LB), Cyclododecanon (CDON), Mittelsieder (MB), Cyclododecanol (CDOL) und Hochsieder (HB);
m) destillatives Abtrennen einer Cyclododecanol-reichen Fraktion (CDOL t.q.) aus dem Oxidationsgemisch (2), welche hinsichtlich Schwersieder (HB) abgereichert ist;
n) Dehydrierung der Cyclododecanol-reichen Fraktion (CDOL t.q.) unter Erhalt des Dehydrierungsgemisches (O).

11. Verfahren nach Anspruch 10, **dadurch gekennzeichnet, dass** die Cyclododecanol (CDOL) und Schwersieder (HB) enthaltenden Fraktionen (C1, C2) in den Schritt m) zurückgeführt wird.

12. Verfahren nach einem der Ansprüche 1 bis 11, **dadurch gekennzeichnet,**
**dass** die Cyclododecanon-reiche Ziel-Fraktion (A) zu Laurinlactam weiterverarbeitet wird und/oder dass das dritte Gemisch (ABC3) thermisch oder stofflich verwertet wird.

## Claims

1. process for removing a cyclododecanone-rich target fraction (A) from a dehydrogenation mixture (O) comprising low boilers (LB), cyclododecanone (CDON), medium boilers (MB), cyclododecanol (CDOL) and high boilers (HB), **characterized by** the following steps:
a) providing the dehydrogenation mixture (0);
b) distillatively removing the low boilers (LB) from the dehydrogenation mixture (0) to obtain a first mixture (ABC1) comprising cyclododecanone (CDON), medium boilers (MB), cyclododecanol (CDOL) and high boilers (HB);
c) feeding the first mixture (ABC1) into a primary side draw column (7);
d) drawing off a first cyclododecanone-rich fraction (A1) from the top of the primary side draw column (7);
e) drawing off a first fraction (C1) comprising cyclododecanol (CDOL) and high boilers (HB) from the bottom of the primary side draw column (7);
f) drawing off a second mixture (ABC2) comprising cyclododecanone (CDON), cyclododecanol (CDOL) and medium boilers (MB) from the side draw of the primary side draw column (7);
g) feeding the second mixture (ABC2) into a secondary side draw column (8);
h) drawing off a second cyclododecanone-rich fraction (A2) from the top of the secondary side draw column (8);
i) drawing off a second fraction (C2) comprising cyclododecanol (CDOL) and high boilers (HB) from the bottom of the secondary side draw column (8);
j) drawing off a third mixture (ABC3) comprising cyclododecanone (CDON), cyclododecanol (CDOL) and medium boilers (MB) from the side draw of the secondary side draw column (8);
k) combining the first cyclododecanone-rich fraction (A1) and the second cyclododecanone-rich fraction (A2) to obtain the cyclododecanone-rich target fraction (A).

2. Process according to Claim 1, **characterized in that** the second mixture (ABC2) is drawn off in liquid form from the primary side draw column and introduced in liquid form into the secondary side draw column.

3. Process according to Claim 2, **characterized in that** the second mixture (ABC2) is drawn off at the separation plane of the primary side draw column at which the liquid concentration of the medium boilers (MB) in the primary side column is at a maximum, and **in that** the second mixture (ABC2) is fed in at a separation plane of the secondary side draw column at which the composition of the second mixture (ABC2) corresponds essentially to the composition of the liquid phase at this separation plane of the primary side draw column.

4. Process according to any of Claims 1 to 3, **characterized in that** the first fraction (C1) comprising cyclododecanol (CDOL) and high boilers (HB) and the second fraction (C2) comprising cyclododecanol (CDOL) and high boilers (HB) are fed in combination to a distillation stage which at least partly removes the high boilers.

5. Process according to any of Claims 1 to 4, **characterized in that** both side draw columns are operated at a pressure below 1 bar absolute, especially below 50 mbar absolute.

6. Process according to Claim 5, **characterized in that** the reduced pressure in the side draw columns is generated by a common vacuum unit.

7. Process according to any of Claims 1 to 6, **characterized in that** the dehydrogenation mixture (0) has the following composition which adds up to 100%:
Low boilers (LB): 1 to 8% by weight,
preferably 3% by weight;
Cyclododecanone (CDON): 60 to 90% by weight,
preferably 70% by weight;
Medium boilers (MB): 0 to 1.5% by weight,
preferably 1% by weight;
Cyclododecanol (CDOL): 10 to 40% by weight,
preferably 24% by weight;
High boilers (HB): 0.1 to 2.5% by weight,
preferably 2% by weight.

8. Process according to any of Claims 1 to 7, **characterized in that** the cyclododecanone-rich target fraction (A) has a cyclododecanone (CDON) content of at least 98% by weight, preferably of 99.5% by weight, and/or **in that** the cyclododecanone-rich target fraction (A) is free of cyclododecanol (CDOL), high boilers (HB) and medium boilers (MB).

9. Process according to any of Claims 1 to 8, **characterized in that** each of the fractions (C1, C2) comprising cyclododecanol (CDOL) and high boilers (HB) is free of cyclododecanone (CDON).

10. Process according to any of Claims 1 to 9, **characterized in that** the providing of the dehydrogenation mixture (0) comprises the following steps:
1) oxidation of cyclododecane (CDAN) with oxygen to obtain an oxidation mixture (2) comprising low boilers (LB), cyclododecanone (CDON), medium boilers (MB), cyclododecanol (CDOL) and high boilers (HB);
m) distillatively removing a cyclododecanol-rich fraction (CDOL t.q.) from the oxidation mixture (2), said fraction having been depleted of high boilers (HB);
n) dehydrogenating the cyclododecanol-rich fraction (CDOL t.q.) to obtain the dehydrogenation mixture (0).

11. Process according to Claim 10, **characterized in that** the fractions (C1, C2) comprising cyclododecanol (CDOL) and high boilers (HB) are recycled into step m).

12. Process according to any of Claims 1 to 11, **characterized in that** the cyclododecanone-rich target fraction (A) is processed further to give laurolactam and/or **in that** the third mixture (ABC3) is utilized thermally or physically.

## Revendications

1. Procédé pour la séparation d'une fraction cible (A) riche en cyclododécanone d'un mélange de déshydrogénation (0) contenant des substances à bas point d'ébullition (LB), de la cyclododécanone (CDON), des substances à point d'ébullition moyen (MB), du cyclododécanol (CDOL) et des substances à point d'ébullition élevé (HB), **caractérisé par** les étapes suivantes, consistant à :
a) préparer le mélange de déshydrogénation (O);
b) séparer par distillation les substances à bas point d'ébullition (LB) du mélange de déshydrogénation (0) avec obtention d'un premier mélange (ABC1) comprenant de la cyclododécanone (CDON), des substances à point d'ébullition moyen (MB), du cyclododécanol (CDOL) et des substances à point d'ébullition élevé (HB) ;
c) injecter le premier mélange (ABC1) dans une colonne de soutirage latéral (7) primaire ;
d) soutirer une première fraction (A1) riche en cyclododécanone en tête de la colonne de soutirage latéral (7) primaire ;
e) soutirer une première fraction (C1) contenant du cyclododécanol (CDOL) et des substances à point d'ébullition élevé (HB) du fond de la colonne de soutirage latéral (7) primaire ;
f) soutirer un deuxième mélange (ABC2) comprenant de la cyclododécanone (CDON), du cyclododécanol (CDOL) et des substances à point d'ébullition moyen (MB) du soutirage latéral de la colonne de soutirage latéral (7) primaire ;
g) injecter le deuxième mélange (ABC2) dans une colonne de soutirage latéral (8) secondaire ;
h) soutirer une deuxième fraction (A2) riche en cyclododécanone en tête de la colonne de soutirage latéral (8) secondaire ;
i) soutirer une deuxième fraction (C2) contenant du cyclododécanol (CDOL) et des substances à point d'ébullition élevé (HB) du fond de la colonne de soutirage latéral (8) secondaire ;
j) soutirer un troisième mélange (ABC3) comprenant de la cyclododécanone (CDON), du cyclododécanol (CDOL) et des substances à point d'ébullition moyen (MB) du soutirage latéral de la colonne de soutirage latéral (8) secondaire ;
k) rassembler la première fraction (A1) riche en cyclododécanone et la deuxième fraction (A2) riche en cyclododécanone avec obtention de la fraction cible (A) riche en cyclododécanone.

2. Procédé selon la revendication 1, **caractérisé en ce que** le deuxième mélange (ABC2) est soutiré sous forme liquide de la colonne de soutirage latéral primaire et introduit sous forme liquide dans la colonne de soutirage latéral secondaire.

3. Procédé selon la revendication 2, **caractérisé en ce que** le deuxième mélange (ABC2) est soutiré au niveau du plan de séparation de la colonne de soutirage latéral primaire sur lequel la concentration liquide des substances à point d'ébullition moyen (MB) dans la colonne latérale primaire est maximale et **en ce que** le deuxième mélange (ABC2) est injecté au niveau d'un plan de séparation de la colonne de soutirage latéral secondaire sur lequel la composition du deuxième mélange (ABC2) correspond essentiellement à la composition de la phase liquide sur ce plan de séparation de la colonne de soutirage latéral primaire.

4. Procédé selon l'une quelconque des revendications 1 à 3, **caractérisé en ce que** la première fraction (C1) contenant du cyclododécanol (CDOL) et des substances à point d'ébullition élevé (HB) et la deuxième fraction (C2) contenant du cyclododécanol (CDOL) et des substances à point d'ébullition élevé (HB) rassemblées sont introduites dans une étape de distillation séparant au moins partiellement les substances à point d'ébullition élevé.

5. Procédé selon l'une quelconque des revendications 1 à 4, **caractérisé en ce que** les deux colonnes de soutirage latéral sont exploitées à une pression inférieure à 1 bar absolu, en particulier inférieure à 50 mbars absolus.

6. Procédé selon la revendication 5, **caractérisé en ce que** la dépression dans les colonnes de soutirage latéral est générée par un appareil de vide commun.

7. Procédé selon l'une quelconque des revendications 1 à 6, **caractérisé en ce que** le mélange de déshydrogénation (0) présente la composition suivante, se complétant à 100% :
Substances à bas point d'ébullition (LB) 1 à 8% en poids, de préférence 3% en poids ;
Cyclododécanone (CDON) : 60 à 90% en poids, de préférence 70% en poids ;
Substances à point d'ébullition moyen (MB) : 0 à 1,5% en poids, de préférence 1% en poids ;
Cyclododécanol (CDOL) : 10 à 40% en poids, de préférence 24% en poids ;
Substances à point d'ébullition élevé (HB) : 0,1 à 2,5% en poids, de préférence 2% en poids.

8. Procédé selon l'une quelconque des revendications 1 à 7, **caractérisé en ce que** la fraction cible (A) riche en cyclododécanone présente une teneur en cyclododécanone (CDON) d'au moins 98% en poids, de préférence de 99,5% en poids et/ou **en ce que** la fraction cible (A) riche en cyclododécanone est exempte de cyclododécanol (CDOL), de substances à point d'ébullition élevé (HB) et de substances à point d'ébullition moyen (MB).

9. Procédé selon l'une quelconque des revendications 1 à 8, **caractérisé en ce que** les fractions (C1, C2) contenant du cyclododécanol (CDOL) et des substances à point d'ébullition élevé (HB) sont à chaque fois exemptes de cyclododécanone (CDON).

10. Procédé selon l'une quelconque des revendications 1 à 9, **caractérisé en ce que** la préparation du mélange de déshydrogénation (0) comprend les étapes suivantes, consistant à :
1) oxyder du cyclododécane (CDAN) avec de l'oxygène avec obtention d'un mélange d'oxydation (2) contenant des substances à bas point d'ébullition (LB), de la cyclododécanone (CDON), des substances à point d'ébullition moyen (MB), du cyclododécanol (CDOL) et des substances à point d'ébullition élevé (HB) ;
m) séparer par distillation une fraction (CDOL q.t.) riche en cyclododécanol du mélange d'oxydation (2) qui est appauvri en ce qui concerne les substances à point d'ébullition élevé (HB) ;
n) déshydrogéner la fraction (CDOL q.t.) riche en cyclododécanol avec obtention du mélange de déshydrogénation (0).

11. Procédé selon la revendication 10, **caractérisé en ce que** les fractions (C1, C2) contenant du cyclododécanol (CDOL) et des substances à point d'ébullition élevé (HB) sont recyclées dans l'étape m).

12. Procédé selon l'une quelconque des revendications 1 à 11, **caractérisé en ce que** la fraction cible (A) riche en cyclododécanone est transformée ultérieurement en lactame laurique et/ou **en ce que** le troisième mélange (ABC3) est valorisé de manière thermique ou matérielle.
